# EUROPEAN PATENT SPECIFICATION

(11) **EP 4 470 975 B1**
(45) Date of publication and mention of the grant of the patent: **28.10.2026**
(21) Application number: 22924134.4
(22) Date of filing: 13.12.2022
(51) Int. Cl.: C01B 33/18, C01B 33/12, C08K 9/02, C08L 101/00, A61K 6/16, A61K 6/17, A61K 6/816, A61K 6/818, A61K 6/76, A61K 6/887, B01J 13/02, C08K 3/22, C08K 3/36, C09C 1/00, C09C 1/30

(54) **METHOD FOR PRODUCING COMPOSITE OXIDE PARTICLES AND CURABLE COMPOSITION**
VERFAHREN ZUR HERSTELLUNG VON VERBUNDOXIDPARTIKELN UND HÄRTBARE ZUSAMMENSETZUNG
MÉTHODE DE PRODUCTION DE PARTICULES D'OXYDE COMPOSITE ET COMPOSITION DURCISSABLE

(30) Priority: 28.01.2022 JP 2022011767
(43) Date of publication of application: 04.12.2024
(73) Proprietor: Tokuyama Dental Corporation, Tokyo 110-0016 (JP)
(72) Inventor: OYA, Naoyuki, Tokyo 110-0016 (JP); MORISAKI, Hiroshi, Tokyo 110-0016 (JP); AKIZUMI, Hironobu, Tokyo 110-0016 (JP)
(74) Representative: HGF
(86) International application number: PCT/JP2022/045838
(87) International publication number: WO 2023/145283

(56) References cited:
- EP-A1- 2 279 723
- EP-A1- 2 463 235
- EP-A1- 3 366 269
- WO-A1-2011/016418
- WO-A1-2017/069274
- JP-A- H11 100 305
- US-B1- 6 417 244
- FLORIAN M WISSER ET AL: "Detection of surface silanol groups on pristine and functionalized silica mixed oxides and zirconia", JOURNAL OF COLLOID AND INTERFACE SCIENCE, ACADEMIC PRESS,INC, US, vol. 374, no. 1, 8 January 2012 (2012-01-08), pages 77 - 82, XP028474987, ISSN: 0021-9797, [retrieved on 20120117], DOI: 10.1016/J.JCIS.2012.01.015

## Description

### TECHNICAL FIELD

The present invention relates to composite oxide particles, a method for producing the same, and a curable composition containing the composite oxide particles.

### BACKGROUND ART

A dental composite resin (hereinafter also referred to as "CR") is one type of material for restoring teeth damaged by dental caries, fractures, or the like, and is formed of a curable composition containing a polymerizable monomer and an inorganic filler and/or an organic filler. Restoration (CR restoration) using the dental composite resin (CR) is rapidly becoming popular because, for example, an amount of tooth substance to be cut can be reduced, a color tone equivalent to that of natural teeth can be provided, and handling thereof is easy.

Natural teeth are composed of dentine and enamel, and have different color tones (hue, chroma, and lightness) at each site. For this reason, in restoration of teeth, it is common to prepare a plurality of CRs in each of which color tone has been adjusted by blending coloring agents such as a pigment or a dye. However, a CR containing a coloring agent as described above may fade or discolor over time after restoration due to aging of the coloring agent, and the appearance of the restored site may not be compatible with the natural teeth in some cases.

Therefore, in recent years, CR (hereinafter, also referred to as "structural coloring CR") has been developed in which structural color developed by an interference action or the like of light is utilized to omit color toning using coloring agents, enabling restoration with high color tone compatibility to natural teeth, which have various color tones (for example, see Patent Document 1). A curable composition described in Patent Document 1 develops structural color by periodically arranging spherical fillers which have uniform particle diameter. In order for the curable composition described in Patent Document 1 to develop structural color, Conditions I and II described below need to be satisfied. Condition I: the spherical filler has an average primary particle diameter within a range of 230 to 1,000 nm, and 90% or more of a total number of spherical filler particles have a primary particle diameter within a range of the average primary particle diameter plus or minus 5%. Condition II: refractive index nF of the spherical filler and refractive index nP of a polymer obtained by polymerizing the polymerizable monomer component satisfy a relationship represented by the following formula:$nP < nF$ (nF and nP each represent refractive index with respect to light having a wavelength of 589 nm at 25°C).

### Citation List

### Patent Document

Patent Document 1: Japanese Patent No. 6250245
Document 2 : EP 2 463 235 A1.

### DISCLOSURE OF THE INVENTION

### Problems to be Solved by the Invention

The curable composition described in Patent Document 1 is very useful because the resulting cured product has high color tone compatibility to natural teeth.

However, as a result of study by the present inventors, it has been found that the curable composition described in Patent Document 1 may be colored after being stored for a long period of time in a paste state before curing, depending on components to be blended. This coloring phenomenon was noticeable when an aliphatic amine compound was blended as a polymerization initiator in order to increase mechanical strength (bending strength) of the obtained cured product. In addition, it has also been found that the curable composition described in Patent Document 1 has low curability after long-term storage, and mechanical strength (bending strength) of the resulting cured product decreases in some cases.

Thus, an object of the present invention is to provide composite oxide particles capable of realizing a curable composition (structural coloring CR) enabling restoration of teeth with high color tone compatibility to natural teeth even after long-term storage and capable of suppressing deterioration of curability even after long-term storage, a method for producing the composite oxide particles, and a curable composition containing the composite oxide particles. Document 2 relates to the preparation of stable 150 nm core-shell particles.

### Means for Solving the Problems

Specific means for solving the above problems include the following aspects. .
.
<1> A method for producing the composite oxide particles consisting of spherical particles having a predetermined average primary particle diameter within a range of 230 to 350 nm,
   in which 90% or more of a total number of the spherical particles have primary particle diameters within a range of the predetermined average primary particle diameter plus or minus 5%,
   each particle constituting the spherical particles is a core-shell particle including a silica-based composite oxide particle
   that includes an oxide of at least one metal element selected from the group consisting of elements in Group 2, elements in Group 3, elements in Group 4, elements in Group 12, and elements in Group 13 of the periodic table and a silica layer that coats a surface of the silica-based composite oxide particle, and
   the silica layer has an average thickness of 3 to 15 nm, the method including:
      a core particle production step including: mixing a solution containing a hydrolysable organosilicon compound and a hydrolysable metal compound containing at least one metal element selected from the group consisting of elements in Group 2, elements in Group 3, elements in Group 4, elements in Group 12, and elements in Group 13 of the periodic table with an alkaline solution to obtain a reaction solution containing core particles,
      a core-shell precursor particle production step including adding a solution containing a hydrolysable organosilicon compound dropwise to the reaction solution containing core particles to obtain a reaction solution containing core-shell precursor particles, and
      a firing step of firing the core-shell precursor particles to obtain the core-shell particles, wherein
      in the core-shell precursor particle production step, the hydrolysable organosilicon compound is added dropwise in an amount of 10 to 60 parts by mass with respect to 100 parts by mass of a total of the hydrolysable organosilicon compound and the hydrolysable metal compound used in the core particle production step, so that a difference ΔD between an average particle diameter D1 of the core particles and an average particle diameter D2 of the core-shell precursor particles is 8 to 40 nm, , and wherein the respective average particle diameter is determined as follows:
         a powder sample is imaged by scanning electron microscopy, an image of the powder is analyzed by image analysis software, the maximum diameter of at least 30 particles visible within a unit field of view of the image is measured, and the average particle diameter is calculated as the arithmetic mean of the measured maximum diameters according to the formula $\overline{x} = \frac{{Σ}_{i = 1}^{n} {x}_{i}}{n}$, wherein n is the number of particles measured and xi is the maximum diameter of the i-th particle.
<2> The method as described in <1>, wherein the hydrolysable metal compound comprises at least one metal element selected from titanium or zirconium.
<3> A method for producing a dental curable composition, the method comprising:
   a step of producing composite oxide particles by the method according to claim 1 or 2; and
   a step of mixing a polymerizable monomer component, the composite oxide particles and a polymerization initiator containing an aliphatic amine compound.
<4> The method as described in <3>, in which refractive index nF of the composite oxide particles with respect to light having a wavelength of 589 nm at 25°C is larger than refractive index nP of a polymer obtained by polymerizing the polymerizable monomer component with respect to light having a wavelength of 589 nm at 25°C.
<5> The curable composition as described in the <6> or <4>, in which the refractive index nF is in a range of 1.45 to 1.58 and the refractive index nP is in a range of 1.40 to 1.57.

### Effects of the Invention

According to the present invention, it is possible to provide composite oxide particles capable of realizing a curable composition (structural coloring CR) enabling restoration of teeth with high color tone compatibility to natural teeth even after long-term storage and capable of suppressing deterioration of curability even after long-term storage, a method for producing the composite oxide particles, and a curable composition containing the composite oxide particles.

### PREFERRED MODE FOR CARRYING OUT THE INVENTION

Hereinafter, specific embodiments to which the present invention is applied will be described in detail.

In the present specification, unless otherwise specified, the expression "x to y" using the numerical values x and y means "x or more and y or less". In this notation, when a unit is attached to only the numerical value y, the unit also shall be applied to the numerical value x.

In the present specification, the term "(meth)acrylic" means both "acrylic" and "methacrylic". Similarly, the term "(meth)acrylate" means both "acrylate" and "methacrylate", and the term "(meth)acryloyl" means both "acryloyl" and "methacryloyl".

### <Composite Oxide Particles>

The composite oxide particles according to the present embodiment consist of spherical particles having a predetermined average primary particle diameter within a range of 230 to 350 nm. In the composite oxide particles of the present embodiment, 90% or more of a total number of the spherical particles have primary particle diameters within a range of the predetermined average primary particle diameter plus or minus 5%, each particle constituting the spherical particles is a core-shell particle including a silica-based composite oxide particle and a silica layer that coats a surface of the silica-based composite oxide particle, and the silica layer has an average thickness of 3 to 15 nm.

By using the composite oxide particles according to the present embodiment, it is possible to obtain a curable composition (structural coloring CR) capable of restoring a natural tooth with high color tone compatibility even after long-term storage and capable of suppressing deterioration of curability even after long-term storage. The reason is not necessarily clear, but is presumed as follows.

In the structural coloring CR, various polymerizable monomers, such as a polymerizable monomer having an aromatic ring structure, are used as the polymerizable monomer component in order to adjust physical properties. Using the polymerizable monomer having an aromatic ring structure tends to improve physical properties of an organic matrix after polymerization, resulting in higher refractive index. In order to cope with the refractive index of such an organic matrix, a silica-based composite oxide particle obtained by combining silica and another metal oxide (for example, alumina, titania, zirconia, and the like) is generally used as the spherical filler. It is known that the silica-based composite oxide has a higher refractive index than that of silica itself and at the same time, a strong acid point (hereinafter, also referred to as "strong acid point") is present on the surface, exhibiting a strong acidic surface state. When such a silica-based composite oxide is blended in a structural coloring CR, the polymerizable monomer component is considered to convert itself or have an unstable curing behavior due to the strong acid point.

In this regard, in the composite oxide particles according to the present embodiment, the surface of the silica-based composite oxide particle is coated with a silica layer, whereby the strong acid point of the surface is reduced. Thus, the composite oxide particles of the present embodiment are considered to be able to suppress coloration or curability deterioration of the structural coloring CR after long-term storage. In addition, when the surface of the silica-based composite oxide particle is simply coated with a silica layer, the color tone compatibility to natural teeth decreases in some cases. In this regard, in the composite oxide particles according to the present embodiment, the average primary particle diameter and the average thickness of the silica layer are appropriately adjusted, and thus, it is considered that deterioration of color tone compatibility can also be suppressed.

The composite oxide particles according to the present embodiment are particularly suitably used as the spherical filler to be blended in the curable composition (structural coloring CR) described in Patent Document 1. The structural coloring CR develops structural color according to the particle diameter of the spherical filler through an interference action or the like of light. Conditions to be satisfied by the curable composition and the spherical filler in order to develop structural color are known (see Patent Document 1). Therefore, in the following, first, the conditions of the structural color development and the relationship between the structural color and the particle diameter will be described, and then, the composite oxide particles according to the present embodiment will be described.

### [Conditions of Structural Color Development and Relationship between Structural Color and Particle Diameter]

As described in Patent Document 1, in order for the cured product of the curable composition (structural coloring CR) to develop structural color, it is necessary to satisfy the following Conditions I and II.

Condition I: the spherical filler has an average primary particle diameter within a range of 230 to 1,000 nm, and 90% or more of a total number of the spherical filler particles have primary particle diameters within a range of the average primary particle diameter plus or minus 5%.

Condition II: refractive index nF of the spherical filler and refractive index nP of a polymer obtained by polymerizing the polymerizable monomer component satisfy a relationship represented by the following formula:

Note that nF and nP can be determined by measuring the refractive index at 25°C with respect to sodium D line (wavelength: 589 nm) using an Abbe refractometer. Hereinafter, in the present specification, the term "refractive index" means refractive index with respect to light having a wavelength of 589 nm at 25°C.

The curable composition described in Patent Document 1 exhibits predetermined structural color when the average primary particle diameter of the spherical filler is in the range of 230 to 1,000 nm and 90% or more of the total number of spherical filler particles have a primary particle diameter in the range of the average primary particle diameter plus or minus 5%. The structural color expressed by interference, diffraction, refraction, scattering, or the like (hereinafter, simply referred to as "interference, scattering, or the like") is developed when diffraction and interference occur according to Bragg's equation: light having a particular wavelength is emphasized, or light other than a light having a particular wavelength is scattered and light having the particular wavelength is reflected, resulting in the development of structural color. The wavelength of light undergoing interference, scattering, and the like depends on the particle diameter of the spherical filler that constitutes the curable composition. For example, when a spherical filler having an average primary particle diameter in the range of 230 to 260 nm is used, the colored light obtained is yellowish, and when a spherical filler having an average primary particle diameter in the range of 260 to 350 nm is used, the colored light obtained is reddish. When a spherical filler having an average primary particle diameter of more than 350 nm is used, blueish, yellowish, and reddish colored lights are periodically obtained.

From the viewpoint of structural color development, 90% or more of the total number of the spherical filler particles preferably have a primary particle diameter in the range of the average primary particle diameter plus or minus 5%, because periodicity of arrangement in the spherical filler is improved.

### [Composite Oxide Particles]

As described above, the composite oxide particles according to the present embodiment consist of spherical particles having a predetermined average primary particle diameter in the range of 230 to 350 nm, and 90% or more of the total number of the spherical particles have primary particle diameters in the range of the predetermined average primary particle diameter plus or minus 5%. According to such composite oxide particles, yellowish-to-reddish clear colored light can be obtained. Note that when the average primary particle diameter of the spherical particles is less than 230 nm, the obtained colored light is blueish and is difficult to match the color tone of the dentine. On the other hand, when the average primary particle diameter of the spherical particles is larger than 350 nm, it becomes difficult to control particle shape or particle diameter in the production method to be described later.

It is sufficient for individual particles constituting the spherical particles to be substantially spherical, and they need not necessarily be perfectly spherical. In general, it is sufficient for the individual particles to have average uniformity of 0.6 or more and preferably 0.8 or more. The average uniformity is obtained by taking a photograph of particles with a scanning electron microscope, measuring the maximum diameter of each particle (30 or more particles) in the unit field of view, and dividing a particle diameter in the direction orthogonal to the maximum diameter by the maximum diameter.

With regard to the average primary particle diameter of the spherical particles and a proportion (hereinafter, also referred to as "existing proportion of average particle diameter particles") of the particles having primary particle diameters in the range of the average primary particle diameter plus or minus 5% in the spherical particles can be determined by methods provided in Examples to be described later.

Each particle constituting the spherical particles is a core-shell particle, in which a surface of a silica-based composite oxide particle (hereinafter, also referred to as "core particle") serving as a core is coated with a silica layer (hereinafter, also referred to as a "shell"). Hereinafter, the core particle and the shell will be described in detail.

### (Core Particles)

The silica-based composite oxide constituting the core particle includes silica and a metal oxide having no absorption band due to d-d transition in visible light wavelengths. The metal oxide preferably contains an oxide of at least one metal element selected from the group consisting of elements in Group 2, elements in Group 3, elements in Group 4, elements in Group 12, and elements in Group 13 in the periodic table. Examples of the metal element belonging to Group 2 of the periodic table include magnesium (Mg), calcium (Ca), strontium (Sr), and barium (Ba). Examples of the metal element belonging to Group 3 of the periodic table include scandium (Sc), yttrium (Y), and the lanthanoid. Examples of the metal element belonging to Group 4 of the periodic table include titanium (Ti), zirconium (Zr), and hafnium (Hf). As the metal element belonging to Group 12 of the periodic table, zinc (Zn), cadmium (Cd), and mercury (Hg) can be mentioned. As the metal element belonging to Group 13 of the periodic table, aluminum (Al), gallium (Ga), and indium (In) can be mentioned. Among these metal elements, at least one metal element selected from the group consisting of Al, Sr, Ba, Ti, Zn, Zr, Hf, La, and Y is preferable, at least one metal element selected from the group consisting of Al, Ti, Zn, Zr, Hf, La, and Y is more preferable, and at least one metal element selected from Ti or Zr is still more preferable.

That is, as the metal oxide capable of forming a composite with silica, Al₂O₃, SrO, BaO, TiO₂, ZnO, ZrO₂, HfO₂, La₂O₃, Y₂O₃, and the like are preferable. Among these, Al₂O₃, TiO₂, ZnO, ZrO₂, HfO₂, La₂O₃, and Y₂O₃ are more preferable because they have a strong acid point and the effect of coating with the silica layer is remarkable. In addition, TiO₂ and ZrO₂ are more preferable because they are easily available as a raw material in the production method to be described later and have good compatibility with a raw material of silica. It is sufficient for the silica-based composite oxide to contain at least one type of these metal oxides, and two or more types thereof may be contained. Examples of the silica-based composite oxide satisfying the above conditions include silica-alumina, silica-titania, silica-zirconia, and silica-titania-zirconia.

Although proportions of the silica and the metal oxide constituting the core particle are not particularly limited, the metal oxide and the silica are preferably contained in a content of 5 to 30 mol% and 70 to 95 mol%, respectively, from the viewpoint of imparting preferable X-ray opacity as the dental curable composition and adjusting the refractive index to a preferable range. It is possible to adjust the refractive index of the obtained core particle to 1.48 to 1.58 by preparing the core particle within this mixing proportion.

The silica-based composite oxide constituting the core particle may further contain an alkali metal oxide in order to suppress influence of the strong acid point. Examples of such an alkali metal oxide include Li₂O and Na₂O. The mixing proportion of the alkali metal oxide in the core particle is preferably 10 mol% or less. Since the alkali metal oxide is not an essential component, the lower limit of the blending proportion of the alkali metal oxide in the core particle is 0 mol%. However, in order to more effectively suppress the influence of the strong acid point, the lower limit is 0.3 mol%.

The average primary particle diameter of core particles is not particularly limited as long as the average primary particle diameter of composite oxide particles falls in the range of 230 to 350 nm, and is preferably in the range of 210 to 340 nm, for example.

### (Shell)

The silica layer constituting the shell covers the strong acid point on the surface of the silica-based composite oxide constituting the core particle, and thereby plays a role of inhibiting an action of the strong acid point on the polymerizable monomer component, the polymerization initiator, and the like when the silica-based composite oxide is blended in the curable composition.

In order to further suppress influence of the strong acid point, the shell may contain an alkali metal oxide in addition to the silica. Examples of the alkali metal oxide include Li₂O and Na₂O. A mixing proportion of the alkali metal oxide in the shell is preferably 5 mol% or less. Since the alkali metal oxide is not an essential component, the lower limit of the mixing proportion of the alkali metal oxide in the shell is 0 mol%.

An average thickness of the shell is 3 to 15 nm. The average thickness of the shell exceeding 15 nm is unsuitable, because blending such composite oxide particles to the structural coloring CR suppresses the structural color development, resulting in bluish white color. The reason why the structural color is suppressed is, though not completely clear, presumed that since the shell has a lower refractive index than the core particle, scattering of light occurs between the shell and the core particle when light is incident, and the scattering intensity becomes equal to or higher than the intensity of the structural color. On the other hand, the average thickness of the shell less than 3 nm is inappropriate, because when the curable composition containing composite oxide particles having such a shell is stored in a paste state before curing for a long period of time, coloring occurs. In order to exhibit the characteristics of suppressing an action of the strong acid point on the polymerizable monomer component, the polymerization initiator, and the like in the curable composition while having the characteristics of structural color, the average thickness of the shell is preferably 5 to 12 nm.

In order to evaluate the average thickness of the shell, for example, the average thickness of the shell may be determined by directly observing the core-shell particles with a transmission electron microscope. Alternatively, the average thickness of the shell may be determined by the following method. First, when producing the composite oxide particles, an average primary particle diameter of a fired product of the core particles and an average primary particle diameter of the core-shell particles are determined by a scanning electron microscope. Next, the average primary particle diameter of the fired product of the core particles is subtracted from the average primary particle diameter of the core-shell particles, followed by division of the obtained value by 2, and the obtained value is defined as the average thickness of the shell. The present inventors measured the average thickness of the shell by both the methods, resulting in no difference in the average thicknesses. Note that the latter method was employed in Examples described below.

### <Method for producing Composite Oxide Particles>

A method for producing the composite oxide particles according to the present embodiment is not particularly limited, but a wet method such as a sol-gel method is preferable from the viewpoint that spherical particles having uniform particle diameter can be easily obtained. A preferred production method includes: a core particle production step of mixing a solution containing a hydrolysable organosilicon compound and a hydrolysable metal compound capable of forming a metal oxide having no absorption band due to d-d transition in visible light wavelengths with an alkaline solution to obtain a reaction solution containing core particles; a core-shell precursor particle production step of adding a solution containing a hydrolysable organosilicon compound dropwise to the reaction solution containing the core particles to obtain a reaction solution containing core-shell precursor particles; and firing the core-shell precursor particles to obtain core-shell particles. Hereinafter, each step will be described in detail.

### [Core Particle Production Step]

In the core particle production step, a solution (hereinafter, also referred to as "hydrolysable raw material solution") containing a hydrolysable organosilicon compound and a hydrolysable metal compound capable of forming a metal oxide having no absorption band due to d-d transition in visible light wavelengths is mixed with an alkaline solution to obtain a reaction solution containing core particles.

### (Hydrolysable Raw Material Solution)

The hydrolysable organosilicon compound is a compound whose functional group covalently bonded to silicon undergoes hydrolysis reaction to form a silanol group. As the hydrolysable organosilicon compound, a silicon alkoxide represented by the formula: Si(OR)₄ (in the formula, R represents an alkyl group) is preferable from the viewpoint of easy availability. In particular, a silicon alkoxide in which R in the formula is a methyl group, an ethyl group, an isopropyl group, or a butyl group is preferable from the viewpoint that reaction control is easy and core particles having a uniform particle diameter are easily obtained. The hydrolysable organosilicon compound does not necessarily need to be present as a monomer, and may contain a condensate (hereinafter, also referred to as a "low condensation product") such as a dimer, a trimer, or the like.

The hydrolysable metal compound is preferably a metal alkoxide or a compound in which one or two of the alkoxy groups of the metal alkoxide are substituted with a carboxy group or a β-dicarbonyl group, from the viewpoint of compatibility with the hydrolysable organosilicon compound. The hydrolysable metal compound preferably contains at least one metal element selected from the group consisting of elements in Group 2, elements in Group 3, elements in Group 4, elements in Group 12, and elements in Group 13 of the periodic table. Preferable examples of the hydrolysable metal compound include Ba(OC₃H₇)₂, Sr(OC₃H₇)₂, Ca(OC₃H₇)₂, Al(OC₃H₇)₃, Y(OC₃H₇)₃, Yb(OC₃H₇)₃, La(OC₃H₇)₃, Ti(OC₃H₇)₄, Ti(OC₄H₉)₄, Zr(OC₄H₉)₄, and Hf(OC₄H₉)₄.

The hydrolysable raw material solution may further contain a hydrolysable alkali metal compound. When the hydrolysable raw material solution contains a hydrolysable alkali metal compound, an alkali metal oxide can be introduced into the core particles. Preferable examples of the hydrolysable alkali metal compound include sodium methoxide, sodium ethoxide, lithium methoxide, and lithium ethoxide.

A solvent of the hydrolysable raw material solution is not particularly limited as long as it dissolves each hydrolysable component, and an alcohol solvent such as methanol, ethanol, isopropanol, butanol, 2-methyl-1-propanol, 2-methyl-2-propanol, ethylene glycol and propylene glycol are suitably used from the viewpoint of reactivity and availability. An ether solvent such as tetrahydrofuran, dioxane, or diethyl ether, or an ester solvent such as ethyl acetate may be mixed with the alcohol solvent.

When preparing the hydrolysable raw material solution, solutions in each of which each hydrolysable component is separately dissolved may be mixed, or to a solution in which a hydrolysable component has been dissolved, another hydrolysable component may be added and dissolved.

A total content of the hydrolysable components in the hydrolysable raw material solution is preferably, for example, 5 to 55% by mass. By adjusting the content to 55% by mass or less, the reaction is easily controlled and spherical particles tend to be easily obtained. Further, by adjusting the content to 5 mass% or more, an amount of solvent used can be reduced.

In order to improve a shape of the core particle to be obtained, it is preferable to add water to the hydrolysable raw material solution. A molar ratio of water to the hydrolysable organosilicon compound is preferably in the range of 0.1 to 1, from the viewpoint that the core particle having the above-described average primary particle diameter can be produced with high productivity.

### (Alkaline Solution)

As the alkaline solution, a solution is used which dissolves the hydrolysable component described above but does not substantially dissolve the silica-based composite oxide particle constituting the core particle.

As the alkaline compound contained in the alkaline solution, ammonia and sodium hydroxide are preferable. The alkali concentration of the alkaline solution is preferably in the range of 1 to 5 mol%, for example.

As the solvent of the alkaline solution, a known organic solvent can be used without particular limitation. The solvent is preferably a water-containing solvent obtained by adding water to an alcohol solvent, an ether solvent, and an ester solvent described above, or a mixed solvent thereof, because such a water-containing solvent has high compatibility with the hydrolysable raw material solution and results in the core particles being obtained with a good shape.

### (Mixing Method)

When the hydrolysable raw material solution and the alkaline solution are mixed, the hydrolysable components undergo hydrolysis and dehydration condensation, resulting in precipitation of core particles. The mixing method is not particularly limited, but a method of continuously adding the hydrolysable raw material solution little by little to an alkaline solution while agitating the solution with a stirring blade, a stirrer, or the like is preferable because the core particles having a uniform particle diameter can be easily obtained. As the mixing time is longer, the shape of the core particle tends to be better, and the mixing time is preferably in the range of 1 to 9 hours from the viewpoint of productivity.

### [Core-Shell Precursor Particle Production Step]

In the core-shell precursor particle production step, a solution (hereinafter, also referred to as "silica coating liquid") containing a hydrolysable organosilicon compound is added dropwise to the reaction solution containing core particles to obtain a reaction solution containing core-shell precursor particles.

The hydrolysable organosilicon compound contained in the silica coating liquid is preferably a silicon alkoxide represented by the formula: Si(OR)₄ (in the formula, R represents an alkyl group), from the viewpoint of industrially easy availability. The hydrolysable organosilicon compound does not necessarily need to exist as a monomer, and may contain a low condensation product.

The silica coating liquid may further contain a hydrolysable alkali metal compound. Since the silica coating liquid contains a hydrolysable alkali metal compound, an alkali metal oxide can be introduced into the shell. Preferable examples of the hydrolysable alkali metal compound include sodium methoxide, sodium ethoxide, lithium methoxide, and lithium ethoxide.

The solvent of the silica coating liquid is preferably an alcohol solvent, an ether solvent, an ester solvent described above, or a mixed solvent thereof, from the viewpoint of compatibility with the reaction solution containing the core particles.

Examples of the addition method of the silica coating liquid include the following method. First, a reaction solution containing core particles is sampled before adding a silica coating liquid dropwise, and an average primary particle diameter D1 of the core particles is measured. Next, a silica coating solution is added dropwise little by little to the reaction solution, sampling is performed at an arbitrary addition time, an average primary particle diameter D2 of the core-shell precursor particles in the reaction solution is measured, and a difference between D2 and D1 is determined, whereby an average degree of formation ΔD of shell of the core-shell precursor particles is evaluated. When ΔD reaches a predetermined value within the range of 8 to 40 nm, the addition is stopped.

The reason why the average thickness of the shells of the composite oxide particles is in the range of 3 to 15 nm, whereas the average degree of formation ΔD of shell is in the range of 8 to 40 nm is due to consideration of shrinkage in the firing step to be described later. In general, wet composite oxide particles synthesized by a sol-gel method include a solvent component or an organic component in the particles or between the particles. When such a residual component is present, the physical properties may be deteriorated, and therefore, it is necessary to fire the composite oxide particles in an aerobic atmosphere to remove the residual component. At this time, it is known that the composite oxide particles shrink due to removal of the solvent and crystallization. In consideration of this shrinkage, by setting the average degree of formation ΔD of shell to within the range of 8 to 40 nm, the average thickness of the shell of the core-shell particles after firing can be adjusted to within the range of 3 to 15 nm.

Though just an empirical value, the content of the hydrolysable organosilicon compound in the silica coating solution is preferably 10 to 60 parts by mass with respect to 100 parts by mass of a total of the hydrolysable organosilicon compound and the hydrolysable metal compound used in the core particle production step. In the range described above, the average degree of formation ΔD of shell can be easily adjusted to within a desired range, and operability can be improved. In this case, sampling and particle diameter measurement during the addition can be omitted. However, sampling during the addition is not excluded.

### [Firing Step]

In the firing step, the core-shell precursor particles are fired to remove solvent molecules and organic components remaining in the core-shell precursor particles and between the core-shell precursor particles, whereby the core-shell particles (composite oxide particles) are obtained. The firing temperature is preferably 700 to 1,500°C, because a denser silica layer can be obtained by desolvation or crystallization. In the firing step, the core-shell precursor particles shrink and become core-shell particles (composite oxide particles). However, a component proportion of the core-shell precursor particles and that of the core-shell particles are the same.

### [Other Steps]

In order to improve affinity with an organic matrix when the obtained composite oxide particles are blended into the structural coloring CR, the composite oxide particles may be subjected to a silane coupling treatment. As the silane coupling agent to be used in the silane coupling treatment, a known silane coupling agent can be used without any limitation.

### <Curable Composition>

The curable composition according to the present embodiment contains a polymerizable monomer component, the composite oxide particles according to the present embodiment described above, and a polymerization initiator. Hereinafter, each component contained in the curable composition according to the present embodiment will be described in detail.

### [Polymerizable Monomer Component]

As the polymerizable monomer component, a radical polymerizable monomer such as a (meth)acrylic compound and a cationic polymerizable monomer such as an epoxide and an oxetane can be used without particular limitation. The polymerizable monomer may be either a monofunctional polymerizable monomer or a polyfunctional polymerizable monomer. Suitable polymerizable monomers include, for example, methyl (meth)acrylate, ethyl (meth)acrylate, n-butyl (meth)acrylate, 2-ethylhexyl (meth)acrylate, (meth)acrylic acid, N-(meth)acryloylglycine, N-(meth)acryloylaspartic acid, N-(meth)acryloyl-5-aminosalicylic acid, 2-hydroxyethyl (meth)acrylate, 3-hydroxypropyl (meth)acrylate, 4-hydroxybutyl (meth)acrylate, 6-hydroxyhexyl (meth)acrylate, 2,2-bis[(3-methacryloyloxy-2-hydroxypropyloxy)phenyl]propane, 2,2-bis(4-methacryloyloxyphenyl)propane, 2,2-bis(4-methacryloyloxypolyethoxyphenyl)propane, triethylene glycol dimethacrylate, tetraethylene glycol dimethacrylate, neopentyl glycol dimethacrylate, trimethylolpropane trimethacrylate, pentaerythritol tetramethacrylate, and the like.

As the polymerizable monomer component, two or more types of polymerizable monomers are generally used in order to adjust the physical properties of a cured product serving as an organic matrix. At this time, from the viewpoint of easily satisfying the relationship with the refractive index to be described later, it is desirable to set the type and mixing amount of the polymerizable monomer so that the refractive index of the polymerizable monomer component (mixture) is in the range of 1.38 to 1.55. That is, when the composite oxide particles according to the present embodiment contain at least one metal oxide selected from titania or zirconia, the refractive index nF thereof is in the range of about 1.45 to 1.58 depending on the silica content, but by setting the refractive index of the polymerizable monomer component in the range of 1.38 to 1.55, the refractive index nP of the obtained polymer can be adjusted to be in the range of about 1.40 to 1.57, which makes it easy to satisfy the condition, nP < nF, for the structural coloring CR to develop the structural color.

### [Composite Oxide Particles]

As the composite oxide particles, the composite oxide particles according to the present embodiment described above are used. In order to develop the structural color, the refractive index nF of the composite oxide particles needs to be higher than the refractive index nP of the polymer obtained by polymerizing the polymerizable monomer component. From the viewpoint of suppressing attenuation of the structural color due to scattering of light, the difference between nF and nP is preferably 0.001 or more, more preferably 0.002 or more, and still more preferably 0.005 or more. Since the structural color is more clearly developed when the transparency of the cured product is high, the difference between nF and nP is preferably 0.1 or less, more preferably 0.05 or less, so that the transparency is not impaired as much as possible.

The composite oxide particles may be blended in the curable composition as a so-called organic-inorganic composite filler, in which an oxide particle is composited with an organic resin. A method for producing the organic-inorganic composite filler is not particularly limited, and for example, a general production method can be employed in which predetermined amounts of respective components including composite oxide particles, a polymerizable monomer component, and a polymerization initiator are mixed and polymerized by a method such as heating or light irradiation, followed by pulverization. Alternatively, the manufacturing method described in WO2011/115007 or WO2013/039169 may be employed. In this production method, inorganic aggregation particles obtained by aggregating composite oxide particles are immersed in a composition containing a polymerizable monomer component, a polymerization initiator, and an organic solvent, the organic solvent is removed, and the polymerizable monomer is polymerized and cured by a method such as heating or light irradiation. The production method described in WO2011/115007 or WO2013/039169 enables obtainment of an organic-inorganic composite filler that comprises: inorganic agglomerated particles comprising agglomerated inorganic primary particles; an organic resin phase that covers the surface of each inorganic primary particle and binds the inorganic primary particles together; and intra-agglomerate voids formed between the organic resin phases that cover the surface of each particle of the inorganic primary particles. As the polymerization initiator, a known polymerization initiator can be used without particular limitation, but since a cured product having a lower yellowness degree can be obtained, a thermal polymerization initiator is preferably used. A thermal polymerization initiator composed of a compound having no aromatic rings in the structure is more preferably used.

The content of the composite oxide particles is preferably 50 to 600 parts by mass with respect to 100 parts by mass of the polymerizable monomer component. When the content of the composite oxide particles is 50 parts by mass or more, colored light due to interference, scattering, or the like is favorably expressed. When composite oxide particles having a refractive index difference of more than 0.1 with respect to the polymer of the polymerizable monomer component are used as the composite oxide particles, the transparency of the cured product may be lowered and the colored light may not be sufficiently expressed. In consideration of these, the content of the composite oxide particles is more preferably 100 to 600 parts by mass, and still more preferably 200 to 600 parts by mass with respect to 100 parts by mass of the polymerizable monomer component.

### [Polymerization Initiator]

As the polymerization initiator, a known polymerization initiator can be used without particular limitation. Among them, it is preferable to contain a photosensitizing compound and an amine compound.

The photosensitizing compound is a compound having a maximum absorption wavelength of 350 to 700 nm and having a function of generating active species effective for polymerization. The active species usually occurs as a result of energy transfer or electron transfer between the photosensitizing compound excited by light absorption and the polymerizable monomer or another compound.

As the photosensitizing compound, a known photosensitizing compound can be used without any limitation. Suitable photosensitizing compounds include, for example, benzoin alkyl ethers such as benzoin methyl ether, benzoin ethyl ether and benzoin isopropyl ether; benzyl ketals such as benzyl dimethyl ketal and benzyl diethyl ketal; benzophenones such as benzophenone, 4,4'-dimethylbenzophenone, and 4-methacryloxybenzophenone; α-diketones such as camphorquinone, 9,10-phenanthrenequinone, benzyl, diacetyl, acetylbenzoyl, 2,3-pentadione, 2,3-octadione, 4,4'-dimethoxybenzyl, acenaphthenequinone, and 9,10-anthraquinone; thioxansone compounds such as 2,4-diethoxythioxansone, 2-chlorothioxansone, and methylthioxansone; bisacylphosphine oxides such as bis-(2,6-dichlorobenzoyl)phenylphosphine oxide, bis-(2,6-dichlorobenzoyl)-2,5-dimethylphenylphosphine oxide, bis-(2,6-dichlorobenzoyl)-4-propylphenylphosphine oxide, bis-(2,6-dichlorobenzoyl)-1-naphthylphosphine oxide, and bis(2,4,6-trimethylbenzoyl)-phenylphosphine oxide; and the like. Among these, α-diketones such as camphorquinone, 9,10-phenanthrenequinone, benzyl, diacetyl, acetylbenzoyl, 2,3-pentadione, 2,3-octadione, 4,4'-dimethoxybenzyl, and acenaphthenequinone are preferable.

The content of the photosensitizing compound is not particularly limited, but is usually 0.01 to 10 parts by mass, preferably 0.03 to 5 parts by mass, and more preferably 0.05 to 2 parts by mass with respect to 100 parts by mass of the polymerizable monomer component.

As the amine compound, known secondary and tertiary amine compounds can be used without any limitation. In particular, from the viewpoint of odor and the like, it is preferable to use an aromatic amine compound. Suitable aromatic amine compounds include N,N-dimethylaniline, N,N-dibenzylaniline, N,N-dimethyl-p-toluidine, N,N-diethyl-p-toluidine, methyl p-(N,N-dimethyl)aminobenzoate, ethyl p-(N,N-dimethyl)aminobenzoate, amyl p-(N,N-dimethyl)aminobenzoate, and the like. In addition, in order to enhance the physical properties of the cured product, an aliphatic amine compound may be used. According to studies by the present inventors, by using the composite oxide particles according to the present embodiment, even when an aliphatic amine compound is used as the amine compound, coloring due to aging can be suppressed. Suitable aliphatic amine compounds include aliphatic tertiary amine compounds such as triethanolamine, N-methyldiethanolamine, triethylamine, tributylamine, N,N-dimethylaminoethyl methacrylate, and N,N-diethylaminoethyl methacrylate.

In the curable composition according to the present embodiment, an aromatic amine compound and an aliphatic amine compound are preferably used in combination because good curability can be obtained. In this case, when the total amount of the amine compound is 100 parts by mass, 20 to 90 parts by mass of the aromatic amine compound and 10 to 80 parts by mass of the aliphatic amine compound are preferable. However, a case where either one of the aromatic amine compound or the aliphatic amine compound is used as the amine compound is not excluded.

The content of the amine compound is not particularly limited, but is usually 10 to 1,000 parts by mass, preferably 50 to 500 parts by mass, and more preferably 75 to 300 parts by mass with respect to 100 parts by mass of the photosensitizing compound.

The curable composition according to the present embodiment may contain a photoacid generator for the purpose of improving curability. Suitable photoacid generators include aryliodonium salt-based compounds, sulfonium salt-based compounds, sulfonic acid ester compounds, s-triazine compounds having a halomethyl group as a substituent, pyridinium salt-based compounds, and the like. The aryliodonium salt-based compounds and s-triazine compounds having a halomethyl group as a substituent are preferred.

Examples of the aryl iodonium salt-based compounds include a salt composed of a cation such as: diphenyl iodonium, bis(p-chlorophenyl) iodonium, ditolyl iodonium, bis(p-methoxyphenyl) iodonium, bis(p-tert-butylphenyl) iodonium, p-isopropylphenyl-p-methylphenyl iodonium, bis(m-nitrophenyl) iodonium, p-tert-butylphenylphenyl iodonium, p-methoxyphenylphenyl iodonium, p-octyloxyphenylphenyliodonium or p-phenoxyphenylphenyliodonium, and an anion such as: nitrate, acetate, chloroacetate, carboxylate or phenolate.

Examples of the s-triazine compound having a halomethyl group as a substituent include an s-triazine compound having at least one of trichloromethyl group or tribromomethyl group.

A content of the photoacid generator is not particularly limited, but is usually 10 to 2,000 parts by mass, preferably 20 to 1,000 parts by mass, and more preferably 50 to 800 parts by mass with respect to 100 parts by mass of the photosensitizing compound.

### [Other Additives]

The curable composition according to the present embodiment may contain other additives such as a polymerization inhibitor and an ultraviolet absorber, as long as the effects thereof are not impaired. For the purpose of viscosity adjustment and the like, the curable composition according to the present embodiment may contain a filler having a particle diameter of less than 0.1 µm, which is sufficiently smaller than the wavelength of light and hardly affects the color tone or transparency.

According to the curable composition of the present embodiment, it is possible to perform restoration that matches the color tone of natural teeth without using a coloring agent such as a pigment. Therefore, in a preferred embodiment, a pigment which may be discolored due to aging deterioration is not blended. However, blending of a pigment itself is not denied, and a pigment may be blended to an extent that does not interfere with the structural color development. Specifically, about 0.0005 to 0.5 parts by mass, preferably about 0.001 to 0.3 parts by mass of the pigment may be added to 100 parts by mass of the polymerizable monomer component.

The curable composition according to the present embodiment is suitably used as a dental filling restorative material, particularly as a dental filling and repairing material represented by a photocurable composite resin, but is not limited thereto, and can be suitably used for other applications. Other applications include, for example, dental cements, restorative materials for abutment construction, and the like.

### EXAMPLES

Hereinafter, the present invention will be described in more detail with reference to Examples, but the present invention is not limited to these Examples.

### <Methods for measuring Various Physical Properties>

Methods for measuring various physical properties in the Examples and the Comparative Examples are as follows.

### (1) Average Primary Particle Diameter of Composite Oxide Particles

A photograph of powder was taken with a scanning electron microscope ("XL-30S", manufactured by Philips N.V.) at a magnification of 5,000 to 100,000 times, the taken image was processed with image analysis software ("IP-1000PC" made by Asahi Kasei Engineering Corp.), the number (30 or more particles) and the particle diameter (maximum diameter) of particles observed within a unit field of view of the photograph were measured and the average primary particle diameter was calculated according to the following equation, based on the measured values.$\overline{x} = \frac{{\sum }_{i = 1}^{n} {x}_{i}}{n} \left(Number average\right)$ **(n: number of particles, xᵢ: primary particle diameter (maximum diameter) of i-th particle)**

### (2) Existing proportion of Average Diameter Particles in Composite Oxide Particles

Among the whole particles (30 or more particles) observed within a unit field of view of the photograph obtained in (1) described above, the number of particles having a primary particle diameter (maximum diameter) outside a particle diameter range of the average primary particle diameter plus or minus 5% was measured and a value thereof was subtracted from the number of the whole particles, whereby the number of particles within the particle diameter range of the average primary particle diameter plus or minus 5% was obtained. According to the following equation: 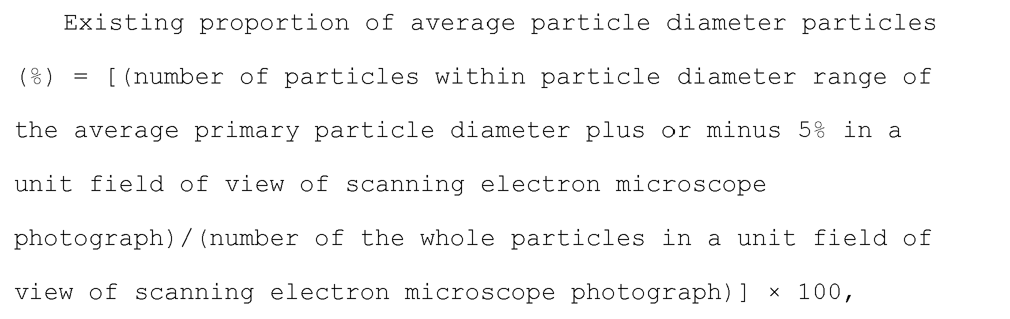 Existing proportion of average particle diameter particles (%) = [(number of particles within particle diameter range of the average primary particle diameter plus or minus 5% in a unit field of view of scanning electron microscope photograph)/(number of the whole particles in a unit field of view of scanning electron microscope photograph)] × 100, the existing proportion of average particle diameter particles was calculated.

### (3) Average Uniformity of Composite Oxide Particles

A photograph of powder was taken with the scanning electron microscope, the number (n: 30 or more) of particles observed within a unit field of view of the photograph and major diameter (Li), which is the maximum diameter of the particle, and minor diameter (Bi), which is a diameter in a direction orthogonal to the major diameter were determined, and the average uniformity was calculated according to the following equation.$Average uniformity = \frac{{\sum }_{i = 1}^{n} Bi / Li}{n}$

### (4) Measurement of Average Degree of Formation ΔD of Silica Layer of Core-Shell Precursor Particles

Photographs of core particles and core-shell precursor particles were taken with a scanning electron microscope, and the average primary particle diameter D1 of the core particles and the average primary particle diameter D2 of the core-shell precursor particles were calculated according to (1) above. According to the following equation: $ΔD = D 2 - D 1$ average degree of formation ΔD of the silica layer of the core-shell precursor particles was calculated.

### (5) Measurement of Average Thickness T of Silica Layers of Composite Oxide Particles

Photographs of a fired product of the core particles and the composite oxide particles were taken with a scanning electron microscope, and an average primary particle diameter D3 of the fired product of the core particles and an average primary particle diameter D4 of the composite oxide particles were calculated according to (1) above. Next, according to the following equation: $T = \left(D4-D3\right) / 2$ an average thickness T of the silica layers of the composite oxide particles was calculated.

### (6) Measurement of Refractive Index

### (6-1) Refractive Index of Polymerizable Monomer Component

The refractive index of the polymerizable monomer (or a mixture of polymerizable monomers) used was measured in a thermostatic chamber at 25°C, by using an Abbe refractometer (manufactured by Atago Co., Ltd.) (measurement wavelength: 589 nm).

### (6-2) Refractive Index of Polymer of Polymerizable Monomer Component

The refractive index of the polymer of the polymerizable monomer (or a mixture of polymerizable monomers) was measured using an Abbe refractometer (manufactured by Atago Co., Ltd.) in a thermostatic chamber at 25°C under substantially the same conditions as the polymerization conditions in the cavity (measurement wavelength: 589 nm).

That is, a uniform mixture containing a polymerizable monomer (or a mixture of polymerizable monomers) and 0.2% by mass of camphorquinone, 0.3% by mass of ethyl p-N,N-dimethylaminobenzoate, and 0.15% by mass of hydroquinone monomethyl ether was placed in a mold having a hole of 7 mmφ × 0.5 mm, and polyester films were pressed against both surfaces of the mold. Thereafter, the resultant mixture was cured by light irradiation for 30 seconds using a halogen-type dental light irradiator ("Demethron LC", manufactured by Sybron) having a light intensity of 500 mW/cm², and then the cured product was taken out of the mold to produce a polymer of the polymerizable monomer. When the polymer was set in the Abbe refractometer (manufactured by Atago Co., Ltd.), in order for the polymer and a measurement surface to be brought into intimate contact with each other, a solvent (bromonaphthalene) which did not dissolve the sample and whose refractive index was higher than that of the sample was added dropwise onto the sample.

### (6-3) Refractive Index of Composite Oxide Particles

The refractive index of the composite oxide particles was measured by a liquid immersion method using an Abbe refractometer (manufactured by Atago Co., Ltd.) (measurement wavelength: 589 nm).

That is, in a thermostatic chamber at 25°C, 1 g of the composite oxide particles was dispersed in 50 mL of anhydrous toluene in a 100 mL sample bottle. While stirring this dispersion with a stirrer, 1-bromotoluene was added dropwise little by little, the refractive index of the dispersion at the time when the dispersion became the clearest was measured, and the obtained value was defined as the refractive index of the composite oxide particles.

### (7) Measurement of Bending Strength

Each of the curable compositions prepared in the Examples and the Comparative Examples was placed in a mold 2 mm wide, 20 mm long, and 2 mm thick having a through hole, and polyester films were pressed against both surfaces of the mold. Thereafter, using a halogen-type dental light irradiator (Optilax LCT manufactured by Kavo Dental Systems Co., Ltd.) having a light intensity of 800 mW/cm², the polymerizable composition was irradiated with light for 20 seconds at a total of three locations in the center and the left and right in the longitudinal direction to cure the curable composition. Then, the mold was turned over and the back surface was also irradiated with light in the same manner. The resulting cured product was removed from the mold and stored in water at 37°C for 24 hours. Thereafter, the cured product was taken out from the water, and the four side surfaces in the longitudinal direction were polished with #1,500 water-resistant polishing paper. The polished cured product was subjected to a three-point bending test using a universal tensile tester autograph (manufactured by Shimadzu Corporation) under the conditions of a distance between supporting points of 20 mm and a crosshead speed of 1 mm/min in the atmosphere at room temperature. The test was performed on 5 test pieces, and an average value was obtained from the obtained results.

### (8) Visual Evaluation of Colored Light

Each of the curable compositions prepared in the Examples and the Comparative Examples was placed in a mold having a hole of 7 mmφ × 1 mm, and polyester films were pressed against both surfaces of the mold. Both surfaces were irradiated with light for 30 seconds using a visible light irradiator (Powerlight Co., Ltd., manufactured by Tokuyama) to cure the polymerizable composition, then the cured product was taken out of the mold and placed on an adhesive surface of a black tape (carbon tape) of about 10 mm square, and the color tone of the colored light was visually confirmed.

### (9) Visual Evaluation of Color Tone Compatibility

The curable composition was filled in a lost portion of a hard resin tooth in which a cavity of class I (diameter of 4 mm and a depth of 4 mm) had been reproduced in the center portion of the occlusal surface of lower right number 6, curing and polishing were performed, and color tone compatibility was visually confirmed. The evaluation criteria are shown below. As the hard resin tooth, a hard resin tooth of high chroma (corresponding to A4) and a hard resin tooth of low chroma (corresponding to A1) in the class of system A (reddish brown) in the shade guide ("VITA Classical", made by Vita) were used.

### - Evaluation criteria -

5: the color tone of the restored product was indistinguishable from that of the hard resin tooth;
4: the color tone of the restored product had good compatibility with the hard resin tooth;
3: the color tone of the restored product was similar to that of the hard resin tooth but was slightly yellow;
2: the color tone of the restored product was similar to that of the hard resin tooth but was not satisfactorily compatible therewith; and
1: the color tone of the restored product was not similar to that of the hard resin tooth.

### (10) Evaluation of Color Tone Compatibility with Colorimeter

The curable composition was filled in a lost portion of a hard resin tooth in which a cavity of class I (diameter of 4 mm and a depth of 4 mm) had been reproduced in the center portion of the occlusal surface of lower right number 6, curing and polishing were performed, and the color tone compatibility was evaluated with a two-dimensional colorimeter ("RC-500" made by PaPaLab Co., Ltd.). As the hard resin tooth, a hard resin tooth of high chroma (corresponding to A4) and a hard resin tooth of low chroma (corresponding to A1) in the class of system A (reddish brown) in the shade guide ("VITA Classical", made by Vita) were used.

The hard resin tooth was set in the two-dimensional colorimeter, an image of the hard resin tooth was taken, image analysis software ("RC Series Image Viewer" made by PaPaLab Co., Ltd.) was used to process the taken image, a color difference (ΔE* in CLELab) between the colorimetric values of the restored portion and the non-restored portion of the hard resin tooth was determined according to the following formula, and with the result, the color tone compatibility was evaluated. Note that L1* refers to lightness index of restored portion of hard resin tooth; a1* and b1* refer to chroma indexes of restored portion of hard resin tooth; L2* refers to lightness index of non-restored portion of hard resin tooth; a2* and b2* refer to chroma indexes of non-restored portion of hard resin tooth; and ΔE* refers to an amount of color tone variation. $ΔE * = {\left\{{\left(ΔL*\right)}^{2}+{\left(Δa*\right)}^{2}+{\left(Δb*\right)}^{2}\right\}}^{1 / 2}$ $ΔL * = L 1 * - L 2 *$ $Δa * = a 1 * - a 2 *$ $Δb * = b 1 * - b 2 *$

### <Production of Composite Oxide Particles>

Abbreviations of compounds used for producing the composite oxide particles are shown below.
TES: tetraethylsilicate
TBZ: tetra(n-butoxy)zirconate
TiPT: tetra(isopropoxy) titanate
NaOMe: 28% by mass sodium methoxide methanol solution

### [Example 1]

4 g of 0.05% by mass hydrochloric acid water and 150 g of TES (manufactured by Colcoat Co., Ltd.) were dissolved in 0.4 L of methanol, and the obtained solution was mixed at room temperature for 2 hours to hydrolyze TES to obtain a mixed solution 1. Subsequently, 44 g of TBZ (manufactured by Nippon Soda Co., Ltd.) and 50 g of 2-methyl-1-propanol were mixed to obtain a mixed solution 2. Then, the mixed solution 2 was added to the mixed solution 1 and stirred for 10 minutes. Subsequently, 5 g of NaOMe was added to the obtained mixed solution while stirring to obtain a hydrolysable raw material solution.

Next, 0.6 L of 2-methyl-1-propanol was filled in a glass reaction vessel with an inner volume of 3 L equipped with a stirrer, and 200 g of a 25% by mass aqueous ammonia solution was added thereto to prepare an ammonia alcohol solution. The hydrolysable raw material solution prepared above was added to this solution over 6 hours while maintaining the temperature of the reaction vessel at 35°C, and after completion of the addition, stirring was continued for 30 minutes to obtain core particles. The reaction solution containing the core particles was sampled, and the average primary particle diameter of the core particles was measured by a scanning electron microscope.

Next, 110 g of TES was dissolved in 0.3 L of methanol to prepare a silica coating liquid. Subsequently, the silica coating solution was added to the reaction solution containing the core particles over 2 hours, and after completion of the addition, stirring was continued for 30 minutes to obtain core-shell precursor particles. The reaction solution containing the core-shell precursor particles was sampled, the average primary particle diameter of the core-shell precursor particles was measured by a scanning electron microscope, and the average degree of formation ΔD of the silica layer was evaluated.

Next, the core-shell precursor particles were collected by suction filtration and dried under reduced pressure at 80°C to obtain white powder. The powder was fired at 800°C for 4 hours to obtain composite oxide particles F1.

The obtained composite oxide particles F1 were subjected to a surface treatment with γ-methacryloyloxypropyltrimethoxysilane to hydrophobize the surface, and then used for preparing a curable composition.

### [Examples 2 to 6 and Comparative Examples 1 to 3]

Composite oxide particles F2 to F9 were obtained in the same manner as in Example 1 except that the raw materials and the amounts used were changed as shown in Table 1. The numerical values in the column of the raw materials in Table 1 represent an amount (unit: g (gram)) of each raw material used.

The obtained composite oxide particles F2 to F9 were subjected to surface treatment with γ-methacryloyloxypropyltrimethoxysilane to hydrophobize the surface, and then used for preparation of a curable composition.

**[Table 1]**

| | | | Core-shell precursor particles | | | | | | Core-shell particles (Composite oxide particles) | | | | | | | | |
|---|---|---|---|---|---|---|---|---|---|---|---|---|---|---|---|---|---|
| | | | Core particle raw material | | | | Shell raw material | Average degree of formation of silica layer ΔD [nm] | Component ratio of core particle portion (mol%) | | | | silica layer thickness T [nm] | Average primary particle diameter [nm] | Refractive index | Existing proportion of average particle diameter particles [%] | Average uniformity |
| | | | TES | TBZ | TIPT | NaOMe | TES | | SiO₂ | ZrO₃ | TiO₂ | Na₃O | | | | | |
| Example | 1 | F1 | 150 | 44 | 0 | 5 | 110 | 38 | 86.5 | 12.0 | 0 | 1.6 | 14 | 250 | 1.515 | 95 | 0.88 |
| | 2 | F2 | 120 | 35 | 0 | 4 | 62 | 21 | 86.5 | 11.9 | 0 | 1.6 | 8 | 230 | 1.515 | 92 | 0.90 |
| | 3 | F3 | 170 | 50 | 0 | 6 | 88 | 26 | 86.4 | 12.0 | 0 | 1.6 | 10 | 280 | 1.515 | 94 | 0.94 |
| | 4 | F4 | 150 | 0 | 44 | 5 | 77 | 24 | 81.8 | 0 | 16.7 | 1.5 | 9 | 310 | 1.535 | 90 | 0.89 |
| | 5 | F5 | 150 | 75 | 0 | 6 | 90 | 27 | 79.5 | 18.8 | 0 | 1.7 | 10 | 286 | 1.542 | 91 | 0.90 |
| | 6 | F6 | 170 | 50 | 0 | 6 | 35 | 11 | 86.5 | 12.0 | 0 | 1.6 | 4 | 266 | 1. 515 | 92 | 0.91 |
| Compara tive Example | 1 | F7 | 150 | 44 | 0 | 5 | 155 | 69 | 86.5 | 12.0 | 0 | 1.6 | 26 | 270 | 1.515 | 91 | 0.86 |
| | 2 | F8 | 150 | 44 | 0 | 5 | 10 | 3 | 86.5 | 12.0 | 0 | 1.6 | 1 | 250 | 1.515 | 90 | 0.89 |
| | 3 | F9 | 100 | 30 | 0 | 3 | 52 | 21 | 86.4 | 12.2 | 0 | 1.4 | 8 | 190 | 1.515 | 90 | 0.95 |

### <Preparation of Curable Compositions>

Abbreviations of the compounds used in the preparation of the curable composition are shown below.

### - Polymerizable monomers -

UDMA: 1,6-bis(methacrylethyloxycarbonylamino)trimethylhexane
3G: triethylene glycol dimethacrylate
Bis-GMA: 2,2-bis[(3-methacryloyloxy-2-hydroxypropyloxy)phenyl]propane
D-2.6E: 2,2-bis(4-methacryloyloxypolyethoxyphenyl)propane

### - Polymerization initiator -

CQ: camphorquinone
MDEOA: methyldiethanolamine
DMBE: ethyl p-(N,N-dimethyl)aminobenzoate
Other ingredients -
HQME: hydroquinone monomethyl ether
DPI: diphenyliodonium-2-carboxylate monohydrate

### [Preparation of Polymerizable Monomer Components]

The polymerizable monomers shown in Table 2 were mixed to prepare polymerizable monomer components M1, M2, and M3. The numerical values in parentheses in Table 2 represent an amount (unit: part by mass) of each polymerizable monomer used.

**[Table 2]**

| | Polymerizable monomer component | Refractive index | |
|---|---|---|---|
| | | Before curing | After curing |
| M1 | UDMA (60) / 3G (40) | 1.474 | 1.509 |
| M2 | bis-GMA (25) / UDMA (25) / 3G (50) | 1.504 | 1.525 |
| M3 | bis-GMA (50) / 3G (50) | 1.506 | 1.540 |

### [Example 7]

To 100 parts by mass of the polymerizable monomer component M1, 0.3 parts by mass of CQ, 0.3 parts by mass of DMBE, 0.3 parts by mass of MDEOA, 0.3 parts by mass of DPI, and 0.15 parts by mass of HQME were added and mixed to prepare a uniform polymerizable monomer composition. Next, the composite oxide particles shown in Table 3 were weighed into a mortar, the polymerizable monomer composition was gradually added under red light, and the obtained mixture was sufficiently kneaded in a dark place to obtain a uniform curable paste. Further, the paste was defoamed under reduced pressure to remove air bubbles, thereby preparing a dental curable composition. Each physical property of the obtained dental curable composition was evaluated based on the above-described method. Component ratios and results are shown in Tables 3 and 4.

### [Examples 8 to 12 and Comparative Examples 4 to 6]

Dental curable compositions were prepared in the same manner as in Example 7 except that the polymerizable monomer components and/or the composite oxide particles were changed as shown in Table 3. Each physical property of the obtained dental curable compositions was evaluated based on the above-described method. Component ratios and results are shown in Tables 3 and 4.

### [Example 13]

A dental curable composition was prepared in the same manner as in Example 7 except that 0.3 parts by mass of DMBE was used as the amine compound. Each physical property of the obtained dental curable composition was evaluated based on the above-described method. Component ratios and results are shown in Tables 3 and 4.

**[Table 3]**

| | | Polymerizable monomer component (parts by mass) | Composite oxide particles (parts by mass) | Photosensi tizing compound (parts by mass) | Amine compound (parts by mass) | Photoacid generator (parts by mass) |
|---|---|---|---|---|---|---|
| Example | 7 | M1 (100) | F1 (230) | CQ (0.3) | DMBE (0.3) / MDEOA (0.3) | DPI (0.3) |
| | 8 | M1 (100) | F2 (230) | CQ (0.3) | DMBE (0.3) / MDEOA (0.3) | DPI (0.3) |
| | 9 | M1 (100) | F3 (230) | CQ (0.3) | DMBE (0.3) / MDEOA (0.3) | DPI (0.3) |
| | 10 | M2 (100) | F4 (230) | CQ (0.3) | DMBE (0.3) / MDEOA (0.3) | DPI (0.3) |
| | 11 | M3 (100) | F5 (230) | CQ (0.3) | DMBE (0.3) / MDEOA (0.3) | DPI (0.3) |
| | 12 | M1 (100) | F6 (230) | CQ (0.3) | DMBE (0.3) / MDEOA (0.3) | DPI (0.3) |
| | 13 | M1 (100) | F1 (230) | CQ (0.3) | DMBE (0.3) | DPI (0.3) |
| Compara tive Example | 4 | M1 (100) | F7 (230) | CQ (0.3) | DMBE (0.3) / MDEOA (0.3) | DPI (0.3) |
| | 5 | M1 (100) | F8 (230) | CQ (0.3) | DMBE (0.3) / MDEOA (0.3) | DPI (0.3) |
| | 6 | M1 (100) | F9 (230) | CQ (0.3) | DMBE (0.3) / MDEOA (0.3) | DPI (0.3) |

**[Table 4]**

| | | Visual evaluation of colored light | Bending strength immediately after the preparation [MPa] | Bending strength after storage at 50° C for one month [MPa] | Color tone compatibility immediately after the preparation (Artificial tooth of system A, reddish-brown) | | | | Color tone compatibility after storage at 50°C for one month (Artificial tooth of system A, reddish-brown) | | | |
|---|---|---|---|---|---|---|---|---|---|---|---|---|
| | | | | | Low chroma | | High chroma | | Low chroma | | High chroma | |
| | | | | | Visual evalua tion | ΔE* | Visual evaluat ion | ΔE* | Visual evalua tion | ΔE* | Visual evalua tion | ΔE* |
| Example | 7 | Red | 125 | 121 | 5 | 0.3 | 5 | 0.5 | 5 | 0.2 | 5 | 0.6 |
| | 8 | Yellow | 120 | 117 | 5 | 0.3 | 4 | 0.8 | 5 | 0.3 | 4 | 0.9 |
| | 9 | Red | 131 | 126 | 5 | 0.3 | 5 | 0.4 | 5 | 0.4 | 5 | 0.3 |
| | 10 | Red | 137 | 136 | 5 | 0.5 | 5 | 0.3 | 4 | 0.8 | 5 | 0.4 |
| | 11 | Red | 128 | 123 | 4 | 0.8 | 5 | 0.2 | 4 | 0.7 | 5 | 0.2 |
| | 12 | Red | 126 | 120 | 4 | 0.7 | 5 | 0.3 | 4 | 0.8 | 5 | 0.3 |
| | 13 | Red | 107 | 105 | 5 | 0.4 | 5 | 0.2 | 5 | 0.3 | 5 | 0.5 |
| Compara tive Example | 4 | White | 127 | 124 | 1 | 3.3 | 1 | 3.5 | 1 | 3.2 | 1 | 3.3 |
| | 5 | Red | 120 | 93 | 5 | 0.2 | 5 | 0.4 | 3 | 1.5 | 3 | 1.2 |
| | 6 | Blue | 119 | 118 | 1 | 3.3 | 1 | 4.0 | 1 | 3.3 | 1 | 4.2 |

As shown in Table 4, the cured product of the dental curable compositions of Examples 7 to 13, which satisfy the conditions of the present invention, exhibited yellow to red structural color, the color tone of the structural color changed little with time, and the mechanical strength of the cured product also changed little with time.

On the other hand, the dental curable composition of Comparative Example 4, which contained composite oxide particles having a silica layer thickness of more than 15 nm, did not exhibit structural color. With the dental curable composition of Comparative Example 5, which contained composite oxide particles having a silica layer thickness of less than 3 nm, the cured product showed good mechanical strength and color tone compatibility immediately after the preparation of the composition, but the mechanical strength and color tone compatibility of the cured product, which was produced after the composition had been stored at 50°C for one month, deteriorated. In addition, the dental curable composition of Comparative Example 6, which contained composite oxide particles having an average primary particle diameter of less than 230 nm, exhibited blueish structural color, and thus was inferior to the dental curable compositions of the Examples in color tone compatibility to the tooth material having a reddish-brown color tone.

## Claims

1. A method for producing a composite oxide particles consisting of spherical particles having a predetermined average primary particle diameter within a range of 230 to 350 nm, wherein 90% or more of a total number of the spherical particles have primary particle diameters within a range of the predetermined average primary particle diameter plus or minus 5%, and each particle constituting the spherical particles is a core-shell particle comprising a silica-based composite oxide particle that comprises an oxide of at least one metal element selected from the group consisting of elements in Group 2, elements in Group 3, elements in Group 4, elements in Group 12, and elements in Group 13 of the periodic table and a silica layer that coats a surface of the silica-based composite oxide particle and has an average thickness of 3 to 15 nm,
the method comprising:
a core particle production step of mixing a solution comprising a hydrolysable organosilicon compound and a hydrolysable metal compound comprising at least one metal element selected from the group consisting of elements in Group 2, elements in Group 3, elements in Group 4, elements in Group 12, and elements in Group 13 of the periodic table with an alkaline solution to obtain a reaction solution comprising core particles,
a core-shell precursor particle production step of adding-a solution comprising a hydrolysable organosilicon compound dropwise to the reaction solution comprising core particles to obtain a reaction solution comprising core-shell precursor particles, and
a firing step of firing the core-shell precursor particles to obtain core-shell particles, wherein
in the core-shell precursor particle production step, the hydrolysable organosilicon compound is added dropwise in an amount of 10 to 60 parts by mass with respect to 100 parts by mass of a total of the hydrolysable organosilicon compound and the hydrolysable metal compound used in the core particle production step, so that a difference ΔD between an average particle diameter D1 of the core particles and an average particle diameter D2 of the core-shell precursor particles is 8 to 40 nm, and
wherein the respective average particle diameter is determined as follows:
a powder sample is imaged by scanning electron microscopy, an image of the powder is analyzed by image analysis software,
the maximum diameter of at least 30 particles visible within a unit field of view of the image is measured, and the average particle diameter is calculated as the arithmetic mean of the measured maximum diameters according to the formula $\overline{x} = \frac{{Σ}_{i = 1}^{n} {x}_{i}}{n} ,$
wherein n is the number of particles measured and xi is the maximum diameter of the i-th particle..

2. The method according to claim 1, wherein
the hydrolysable metal compound comprises at least one metal element selected from titanium or zirconium.

3. A method for producing a dental curable composition, the method comprising:
a step of producing composite oxide particles by the method according to claim 1 or 2; and
a step of mixing a polymerizable monomer component containing a (meth)acrylic compound, the composite oxide particles, and a polymerization initiator containing an aliphatic amine compound.

4. The method for producing the dental curable composition according to claim 3, wherein refractive index nF of the composite oxide particles with respect to light having a wavelength of 589 nm at 25°C is larger than refractive index nP of a polymer obtained by polymerizing the polymerizable monomer component with respect to light having a wavelength of 589 nm at 25°C.

5. The method for producing the dental curable composition according to claim 4, wherein the refractive index nF is in a range of 1.45 to 1.58 and the refractive index nP is in a range of 1.40 to 1.57.

## Patentansprüche

1. Verfahren zur Herstellung von Verbundoxidpartikeln, die aus sphärischen Partikeln mit einem vorbestimmten mittleren Primärpartikeldurchmesser in einem Bereich von 230 bis 350 nm bestehen, wobei 90 % oder mehr der Gesamtzahl der sphärischen Partikel Primärpartikeldurchmesser innerhalb eines Bereichs des vorbestimmten mittleren Primärpartikeldurchmessers ± 5 % aufweisen und jedes die sphärischen Partikel bildende Partikel ein Kern-Schale-Partikel ist, das ein siliciumdioxidbasiertes Verbundoxidpartikel, welches ein Oxid mindestens eines Metallelements umfasst, das aus der Gruppe ausgewählt ist, die aus Elementen der Gruppe 2, Elementen der Gruppe 3, Elementen der Gruppe 4, Elementen der Gruppe 12 und Elementen der Gruppe 13 des Periodensystems besteht, sowie eine Siliciumdioxidschicht umfasst, die eine Oberfläche des siliciumdioxidbasierten Verbundoxidpartikels beschichtet und eine mittlere Dicke von 3 bis 15 nm aufweist,
wobei das Verfahren umfasst:
einen Kernpartikel-Herstellungsschritt des Mischens einer Lösung, die eine hydrolysierbare Organosiliciumverbindung und eine hydrolysierbare Metallverbindung umfasst, welche mindestens ein Metallelement umfasst, das aus der Gruppe ausgewählt ist, die aus Elementen der Gruppe 2, Elementen der Gruppe 3, Elementen der Gruppe 4, Elementen der Gruppe 12 und Elementen der Gruppe 13 des Periodensystems besteht, mit einer alkalischen Lösung, um eine Reaktionslösung zu erhalten, die Kernpartikel umfasst,
einen Kern-Schale-Vorläuferpartikel-Herstellungsschritt des tropfenweisen Zugebens einer Lösung, die eine hydrolysierbare Organosiliciumverbindung umfasst, zu der Reaktionslösung, die Kernpartikel umfasst, um eine Reaktionslösung zu erhalten, die Kern-Schale-Vorläuferpartikel umfasst, und
einen Ausheizschritt des Ausheizens der Kern-Schale-Vorläuferpartikel, um Kern-Schale-Partikel zu erhalten, wobei
im Kern-Schale-Vorläuferpartikel-Herstellungsschritt die hydrolysierbare Organosiliciumverbindung in einer Menge von 10 bis 60 Masseteilen bezogen auf 100 Masseteile der Gesamtmenge der im Kernpartikel-Herstellungsschritt verwendeten hydrolysierbaren Organosiliciumverbindung und hydrolysierbaren Metallverbindung tropfenweise zugegeben wird, sodass eine Differenz ΔD zwischen einem mittleren Partikeldurchmesser D1 der Kernpartikel und einem mittleren Partikeldurchmesser D2 der Kern-Schale-Vorläuferpartikel 8 bis 40 nm beträgt, und
wobei der jeweilige mittlere Partikeldurchmesser wie folgt bestimmt wird:
eine Pulverprobe wird mittels Rasterelektronenmikroskopie abgebildet, ein Bild des Pulvers wird mittels Bildanalysesoftware analysiert, der maximale Durchmesser von mindestens 30 Partikeln, die innerhalb eines Einheitssichtfelds des Bildes sichtbar sind, wird gemessen, und der mittlere Partikeldurchmesser wird als arithmetisches Mittel der gemessenen maximalen Durchmesser gemäß der Formel $\overline{x} = \frac{{\sum }_{i = 1}^{n} {x}_{i}}{n}$ berechnet,
wobei n die Anzahl der gemessenen Partikel und xi der maximale Durchmesser des i-ten Partikels ist.

2. Verfahren nach Anspruch 1, wobei
die hydrolysierbare Metallverbindung mindestens ein aus Titan und Zirkonium ausgewähltes Metallelement umfasst.

3. Verfahren zur Herstellung einer härtbaren Dentalzusammensetzung, wobei das Verfahren umfasst:
einen Schritt, bei dem Verbundoxidpartikel nach dem Verfahren gemäß Anspruch 1 oder 2 hergestellt werden; und
einen Schritt, bei dem eine polymerisierbare Monomerkomponente, die eine (Meth)acrylverbindung enthält, die Verbundoxidpartikel und ein Polymerisationsinitiator, der eine aliphatische Aminverbindung enthält, vermischt werden.

4. Verfahren zur Herstellung der härtbaren Dentalzusammensetzung nach Anspruch 3, wobei der Brechungsindex nF der Verbundoxidpartikel für Licht mit einer Wellenlänge von 589 nm bei 25 °C größer ist als der Brechungsindex nP eines durch Polymerisation der polymerisierbaren Monomerkomponente erhaltenen Polymers für Licht mit einer Wellenlänge von 589 nm bei 25 °C.

5. Verfahren zur Herstellung der härtbaren Dentalzusammensetzung nach Anspruch 4, wobei der Brechungsindex nF in einem Bereich von 1,45 bis 1,58 liegt und der Brechungsindex nP in einem Bereich von 1,40 bis 1,57 liegt.

## Revendications

1. Procédé de production de particules d'oxyde composite constituées de particules sphériques ayant un diamètre moyen prédéterminé de particule primaire dans une plage de 230 à 350 nm, 90 % ou plus du nombre total des particules sphériques présentant des diamètres de particule primaire compris dans une plage de ± 5 % par rapport audit diamètre moyen prédéterminé de particule primaire, et chaque particule constituant les particules sphériques étant une particule cœur-coquille comprenant une particule d'oxyde composite à base de silice comprenant un oxyde d'au moins un élément métallique sélectionné dans le groupe constitué des éléments du groupe 2, des éléments du groupe 3, des éléments du groupe 4, des éléments du groupe 12 et des éléments du groupe 13 du tableau périodique, ainsi qu'une couche de silice revêtant une surface de la particule d'oxyde composite à base de silice et ayant une épaisseur moyenne de 3 à 15 nm,
le procédé comprenant :
une étape de production de particules de cœur consistant à mélanger une solution comprenant un composé organosilicié hydrolysable et un composé métallique hydrolysable comprenant au moins un élément métallique sélectionné dans le groupe constitué des éléments du groupe 2, des éléments du groupe 3, des éléments du groupe 4, des éléments du groupe 12 et des éléments du groupe 13 du tableau périodique avec une solution alcaline afin d'obtenir une solution réactionnelle comprenant des particules de cœur,
une étape de production de particules précurseurs cœur-coquille consistant à ajouter goutte à goutte une solution comprenant un composé organosilicié hydrolysable à la solution réactionnelle comprenant des particules de cœur afin d'obtenir une solution réactionnelle comprenant des particules précurseurs cœur-coquille, et
une étape de cuisson consistant à cuire les particules précurseurs cœur-coquille afin d'obtenir des particules cœur-coquille, dans laquelle,
lors de l'étape de production de particules précurseurs cœur-coquille, le composé organosilicié hydrolysable est ajouté goutte à goutte en une quantité de 10 à 60 parties en masse pour 100 parties en masse de la quantité totale du composé organosilicié hydrolysable et du composé métallique hydrolysable utilisés lors de l'étape de production de particules de cœur, de sorte qu'une différence ΔD entre un diamètre moyen de particule D1 des particules de cœur et un diamètre moyen de particule D2 des particules précurseurs cœur-coquille soit de 8 à 40 nm, et
le diamètre moyen de particule correspondant étant déterminé comme suit :
un échantillon de poudre est observé par microscopie électronique à balayage, une image de la poudre est analysée au moyen d'un logiciel d'analyse d'images, le diamètre maximal d'au moins 30 particules visibles dans un champ de vision unitaire de l'image est mesuré, et le diamètre moyen de particule est calculé en tant que moyenne arithmétique des diamètres maximaux mesurés selon la formule $\overline{x} = \frac{{\sum }_{i = 1}^{n} {x}_{i}}{n}$
dans laquelle n représente le nombre de particules mesurées et xi représente le diamètre maximal de la particule d'indice i.

2. Le procédé selon la revendication 1, dans lequel
le composé métallique hydrolysable comprend au moins un élément métallique sélectionné parmi le titane et le zirconium.

3. Procédé de production d'une composition dentaire durcissable, le procédé comprenant :
une étape dans laquelle des particules d'oxyde composite sont produites par le procédé selon la revendication 1 ou 2 ; et
une étape dans laquelle un composant monomère polymérisable contenant un composé (méth)acrylique, les particules d'oxyde composite et un initiateur de polymérisation contenant un composé amine aliphatique sont mélangés.

4. Le procédé de production de la composition dentaire durcissable selon la revendication 3, dans lequel l'indice de réfraction nF des particules d'oxyde composite pour une lumière ayant une longueur d'onde de 589 nm à 25 °C est supérieur à l'indice de réfraction nP d'un polymère obtenu par polymérisation du composant monomère polymérisable pour une lumière ayant une longueur d'onde de 589 nm à 25 °C.

5. Le procédé de production de la composition dentaire durcissable selon la revendication 4, dans lequel l'indice de réfraction nF est compris dans une plage de 1,45 à 1,58 et l'indice de réfraction nP est compris dans une plage de 1,40 à 1,57.
